# EUROPEAN PATENT APPLICATION

(11) **EP 4 516 291 A1**
(43) Date of publication of application: **05.03.2025**
(21) Application number: 22965619.4
(22) Date of filing: 29.11.2022
(51) Int. Cl.: A61K 9/19, A61K 9/20, A61K 41/00, A61P 35/00, A61J 3/00, A61J 3/02

(54) **AMINOLEVULINIC ACID HYDROCHLORIDE FREEZE-DRIED FORMULATION AND PREPARATION METHOD THEREFOR**

(30) Priority: 17.11.2022 CN 202211440166
(71) Applicant: Zhaoke Pharmaceutical (Hefei) Co., Ltd, Hefei, Anhui 230088 (CN)
(72) Inventor: LIU, Jing, Hefei, Anhui 230088 (CN); LI, Gang, Hefei, Anhui 230088 (CN); LING, Wei, Hefei, Anhui 230088 (CN); LI, Xiaoyi, Hefei, Anhui 230088 (CN); DAI, Xiangrong, Hefei, Anhui 230088 (CN); YIN, Lei, Hefei, Anhui 230088 (CN)
(74) Representative: Isern Patentes y Marcas S.L.
(86) International application number: PCT/CN2022/134834
(87) International publication number: WO 2024/103440

(57) **Abstract**

Provided are an aminolevulinic acid hydrochloride freeze-dried formulation and a preparation method thereof. With the character, moisture, related substances, and content of the aminolevulinic acid hydrochloride freeze-dried formulation as evaluation indicators, the processes such as temperature, rate, time, repeated freezing and thawing in the pre-freezing and sublimation process are explored. Meanwhile, the amplified freeze-drying process is optimized, and the character, appearance, and quality of the finished product after freeze drying all meet the requirements.

## Description

### Technical field

The present invention belongs to the technical field of pharmaceutical preparations, and specifically relates to an aminolevulinic acid hydrochloride freeze-dried formulation and a preparation method thereof.

### Background technology

5-aminolevulinic acid (5-ALA), also known as 5-amino-4-ketovaleric acid, has a molecular formula of C₅H₉NO₃, a molecular weight of 131.13, and a chemical structure of 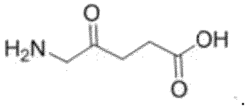 5-ALA is a prodrug and a natural amino acid precursor in the synthesis of hemoglobin. It does not have photosensitivity itself but can produce protoporphyrias IX (PPIX) with strong photosensitivity under the action of various enzymes in the cell mitochondria. PPIX can bind Fe²⁺ and convert into heme under the action of ferrochelatase. The level of ferrochelatase in tumor cells is downregulated, and the uptake of 5-ALA by tumor cells is increased due to the destruction of the blood-brain barrier, the increased neovascularization, and the overexpression of membrane transporters in glioma cells. As a result, after a large amount of exogenous 5-ALA is given, PPIX selectively aggregates in tumor cells. After irradiation with excitation light of about 400 nm, PPIX emits strong purple-red fluorescence (about 600 nm). By giving this drug for photodynamic diagnosis before surgery, the fluorescence coloration of the tumor site in the surgical field can be visualized, thereby distinguishing malignant tumor tissue from normal tissue and achieving the purpose of safe tumor removal to the maximum extent. Therefore, 5-ALA is often used as a photosensitive diagnostic drug in clinical practice.

Aminolevulinic acid hydrochloride is the hydrochloride of 5-ALA (5-ALA HCl), with a molecular weight of 167.59, and is currently the main application form of 5-ALA. 5-ALA HCl has good water solubility and strong hygroscopicity, but has poor stability when present in a solution state and is easy to degrade and produce impurities. If it is developed into a freeze-dried formulation, the stability of the formulation can be improved, which is convenient for transportation and storage. The advantages of freeze drying can be summarized as follows:
1. Drying under low temperature conditions can reduce the degradation of heat-sensitive products;
2. Accurate feeding can be performed in a liquid state;
3. The moisture content of the final product can be controlled during the process;
4. The freeze-dried product can have a better physical shape.

However, since aminolevulinic acid hydrochloride is a hydrochloride, the concentration of the drug solution is very high, and there are no excipients in the formulation, resulting in dense crystal nuclei formed during the pre-freezing process. At the same time, the eutectic point and collapse temperature of the drug solution are low, which is not conducive to the sublimation of water, thereby prone to cause the freeze-dried sample to float, partially dissolve, and collapse, affecting the appearance and quality of the product and bringing great challenges to freeze drying.

In view of this, this invention is specially proposed.

### Content of the invention

In order to develop a freeze-drying process for aminolevulinic acid hydrochloride, solve the appearance and quality problems of its freeze-dried formulations, and obtain products with satisfactory appearance and quality, the present invention provides the following technical solutions:
In the first aspect of the present invention, a method for preparing a freeze-dried formulation of aminolevulinic acid hydrochloride is provided, which includes the following steps:
S1, preparing an aminolevulinic acid hydrochloride solution, specifically including: adding 70-90% of the prescribed amount of injection water into a container, preferably, adding 80% of the injection water, starting stirring, adding the prescribed amount of aminolevulinic acid hydrochloride; adding injection water to the full amount, starting stirring, stopping stirring after complete dissolution, filtering through a filter device below 0.22 µm, preferably, sterilizing with a 0.22 µm filter sterilizer; the final concentration of aminolevulinic acid hydrochloride is 100-200 mg/mL, preferably 150 mg/mL;
S2, filling, specifically, filling the solution obtained in (1) into a 50 mL vial at a standard of 7.5-15 mL/bottle, preferably, filling at a standard of 10 mL/bottle, and stoppering (partially stoppering).
S3, freeze drying, which includes:
   S3.1, pre-freezing: lower the temperature to -45°C or below at 5.0-15.0 °C/h, and keep it for 10-20 h after the temperature drops to -45°C;
   S3.2, vacuuming: vacuuming to a pressure of ≤50 pa;
   S3.3, first sublimating: raise the temperature to -15°C or below at 0.5-5.0 °C/h, and keep it for 100-150 h after the temperature rises to -15°C;
   S3.4, second sublimating: raise the temperature to 35°C or below at 1.0-10.0 °C/h, and keep it for 6-10 h after the temperature rises to 35°C;
   S3.5, stoppering: after the pressure rise test is completed, nitrogen is introduced, the pressure is set to 0.1-1.0 BarA, and vacuum stoppering is performed. After the stoppering, the plate layer is raised and the gas is introduced to restore the normal pressure to obtain the freeze-dried product.

Preferably, the subpackaging is performed by a filling machine under Class A laminar flow.

Preferably, the freeze-dried product can be sealed by capping.

Optionally, the freeze-dried formulation can be a freeze-dried powder or a freeze-dried tablet.

Optionally, the freeze-dried formulation includes a sterile freeze-dried formulation.

The second aspect of the present invention provides a freeze-dried formulation of aminolevulinic acid hydrochloride prepared by the preparation method of the freeze-dried formulation of aminolevulinic acid hydrochloride described in the first aspect of the present invention.

Preferably, the moisture content of the freeze-dried formulation of aminolevulinic acid hydrochloride is ≤1.0% and the related substances are ≤1.0%.

Further, the related substances include known impurities and unknown impurities. The content of the known impurities in the freeze-dried formulation of aminolevulinic acid hydrochloride does not exceed 0.1%, and the known impurities are impurity A and mesityl oxide, and the impurity A is 3,3'-(pyrazine-2,5-diyl)dipropanoic acid, and its structural formula is

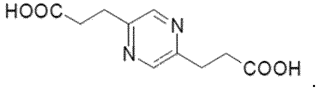

Preferably, the content of the component with the largest proportion of the unknown impurities in the aminolevulinic acid hydrochloride freeze-dried formulation does not exceed 0.2%.

Preferably, the content of the effective constituent in the freeze-dried formulation is 95%-105% of the labeled amount, and the effective constituent is aminolevulinic acid hydrochloride.

Preferably, the pH of the freeze-dried formulation is 2.0-3.0.

Preferably, the reconstitution time of the freeze-dried formulation is ≤2 min.

Optionally, the freeze-dried formulation is a freeze-dried powder or a freeze-dried tablet.

Optionally, the freeze-dried formulation also contains pharmaceutically acceptable excipients.

The beneficial effects of the present invention are as follows:
The present invention investigates the effects of different concentrations of aminolevulinic acid hydrochloride solutions and different filling volumes on freeze drying and determines that the filling volume is 7.5-15 mL and the concentration is 100-200 mg/mL is more appropriate.

The present invention uses the pharmaceutical character, moisture content, and related substances of aminolevulinic acid hydrochloride as evaluation indicators, explores the processes such as temperature, rate, time, and repeated freezing and thawing in the pre-freezing and sublimation processes, and optimizes the amplified freeze-drying process. The results show that the freeze-drying process provided by the present invention can significantly solve the problems of floating, re-dissolving, and collapse of the drug cake in the freeze-drying process, and the character, appearance, and quality of the freeze-dried finished product all meet the requirements.

### Specific implementations

In order to more clearly explain the technical solution of the present invention, the technical solution of the present invention will be further explained below in conjunction with specific implementations:

### Example 1 Preparation process and quality control of aminolevulinic acid hydrochloride freeze-dried powder

### 1. Freeze-drying process

### (1) Preparation of aminolevulinic acid hydrochloride solution

80% of the prescribed amount of injection water is added into the preparation tank, start stirring, the prescribed amount of aminolevulinic acid hydrochloride is added (3.3 kg); injection water is added to the full amount (33 L), start stirring, and stop stirring after a complete dissolution. A sterilization by a filtration is performed through a 0.22 µm filter, waiting for filling.

### (2) Filling

Under Class A laminar flow, aminolevulinic acid hydrochloride solution is filled into 50 mL vials through a filling machine, with a standard filling volume of 15 mL/bottle. Stoppering and partially stoppering are conducted on the filled 50 mL vial, and it is put into the box for freeze drying.

### (3) Freeze drying

Pre-freezing: the temperature is lowered to -45°C or below at 5.0 °C/h, and kept for 10 h after the temperature drops to -45°C.

The vacuum pump is turned on to vacuum, and the sublimation is started when the vacuum control is ≤50 pa.

First sublimation stage: the temperature is raised to -15°C or below at 0.5 °C/h, and kept for 100 h after the temperature rises to -15°C.

Secondary sublimation stage: the temperature is raised to 35°C or below at 1.0 °C/h and kept for 6 h after the temperature rises to 35°C.

Stoppering and transferring: after the pressure rise test, nitrogen is introduced, and the pressure is set to 0.1 BarA, and vacuum stoppering is performed. After stoppering, the plate layer is raised and the gas is introduced to restore the normal pressure, and then the product is transferred.

### (4) Capping

The freeze-dried product is sealed by capping.

The appearance of the sample prepared in this example does not appear to float, collapse, and redissolve.

### Example 2 Preparation process and quality control of aminolevulinic acid hydrochloride freeze-dried powder

### 1. Freeze-drying process

### (1) Preparation of aminolevulinic acid hydrochloride solution

80% of the prescribed amount of injection water is added into the preparation tank, start stirring, the prescribed amount of aminolevulinic acid hydrochloride is added (3.3 kg); injection water is added to the full amount (16.5 L), start stirring, and stop stirring after a complete dissolution. A sterilization by a filtration is performed through a 0.22 µm filter, waiting for filling.

### (2) Filling

Under Class A laminar flow, aminolevulinic acid hydrochloride solution is filled into 50 mL vials through a filling machine, with a standard filling volume of 7.5 mL/bottle. Stoppering and partially stoppering are conducted on the filled 50 mL vial, and it is put into the box for freeze drying.

### (3) Freeze drying

Pre-freezing: the temperature is lowered to -45°C or below at 5.0 °C/h, and kept for 20 h after the temperature drops to -45°C.

The vacuum pump is turned on to vacuum, and the sublimation is started when the vacuum control is ≤50 pa.

First sublimation stage: the temperature is raised to -15°C or below at 5.0 °C/h, and kept for 150 h after the temperature rises to -15°C.

Secondary sublimation stage: the temperature is raised to 35°C or below at 10.0 °C/h and kept for 10 h after the temperature rises to 35°C.

Stoppering and transferring: after the pressure rise test, nitrogen is introduced, and the pressure is set to 1.0 BarA, and vacuum stoppering is performed. After stoppering, the plate layer is raised and the gas is introduced to restore the normal pressure, and then the product is transferred.

### (4) Capping

The freeze-dried product is sealed by capping.

The appearance of the sample prepared in this example does not appear to float, collapse, and redissolve.

### Example 3 Testing of the aminolevulinic acid hydrochloride freeze-dried powder prepared in Example 1 and Example 2

The test results of the prepared aminolevulinic acid hydrochloride freeze-dried powder are shown in Table 1, and all indicators meet the requirements.

**Table 1 Indicators of the aminolevulinic acid hydrochloride freeze-dried powder prepared in Example**

| Batch number | pH | Reconstitution time | Moisture | Related substances | Content (%) |
|---|---|---|---|---|---|
| 20210812 | 2.7 | comply with regulations | 0.27% | comply with regulations | 99.1% |
| 20210928 | 2.7 | comply with regulations | 0.28% | comply with regulations | 98.3% |

### Example 4 Preparation process and quality control of aminolevulinic acid hydrochloride freeze-dried powder

### 1. Freeze-drying process

### (1) Preparation of aminolevulinic acid hydrochloride solution

80% of the prescribed amount of injection water is added into the preparation tank, start stirring, the prescribed amount of aminolevulinic acid hydrochloride is added (3.3 kg); injection water is added to the full amount (22 L), start stirring, and stop stirring after a complete dissolution. A sterilization by a filtration is performed through a 0.22 µm filter, waiting for filling.

### (2) Filling

Under Class A laminar flow, aminolevulinic acid hydrochloride solution is filled into 50 mL vials through a filling machine, with a standard filling volume of 10 mL/bottle. Stoppering and partially stoppering are conducted on the filled 50 mL vial, and it is put into the box for freeze drying.

### (3) Freeze drying

Pre-freezing: the temperature is lowered to -40°C or below at 5.0 °C/h, and kept for 20 h after the temperature drops to -40°C.

The vacuum pump is turned on to vacuum, and the sublimation is started when the vacuum control is 100 pa.

First sublimation stage: the temperature is raised to -15°C at 5.0 °C/h, and kept for 70 h after the temperature rises to -15°C.

Secondary sublimation stage: the temperature is raised to 35°Cat 10.0 °C/h and kept for 4 h after the temperature rises to 35°C.

Stoppering and transferring: after the pressure rise test, nitrogen is introduced, and the pressure is set to 1.0 BarA, and vacuum stoppering is performed. After stoppering, the plate layer is raised and the gas is introduced to restore the normal pressure, and then the product is transferred.

### (4) Capping

The freeze-dried product is sealed by capping.

The appearance of the sample prepared in this example appears to float, collapse, and redissolve.

No relevant patents for the freeze-drying technology of this product have been retrieved in the prior art. This technology can produce sterile freeze-dried formulations, solve the problems of floating, re-dissolution, and collapse of the drug cake during the freeze-drying process, and the character, appearance, and quality of the freeze-dried finished product all meet the requirements, thereby improving the stability of this product.

## Claims

1. A preparation method of an aminolevulinic acid hydrochloride freeze-dried formulation, comprising the following steps:
S1, preparing an aminolevulinic acid hydrochloride solution, specifically comprising: adding 70%-90% of a prescribed amount of an injection water into a container, starting a stirring, and adding a prescribed amount of aminolevulinic acid hydrochloride; adding the injection water to a full amount, starting the stirring, stopping the stirring after a complete dissolution, and performing a sterilization by a filtration through a filter below 0.22 µm; wherein a concentration of the aminolevulinic acid hydrochloride solution is 100-200 mg/mL;
S2, filling, specifically comprising filling a solution obtained in the step (1) into a 50 mL vial at a standard of 7.5-15 mL/bottle, and partially stoppering;
S3, freeze drying, comprising the following steps in sequence:
S3.1, pre-freezing: lowering a temperature of a product obtained in the step 2 to -45°C or below at a rate of 5.0-15.0 °C/h, and keeping it for 10-20 h after the temperature drops to -45°C;
S3.2, vacuuming: vacuuming a product obtained in a pre-freezing step, and controlling a vacuum pressure to be equal to or less than 50 pa;
S3.3, first sublimating: raising a temperature of a product obtained in a vacuuming step to - 15°C or below at a rate of 0.5-5.0 °C/h, and keeping it for 100-150 h after the temperature rises to -15°C;
S3.4, second sublimating: raising a temperature of a product obtained in a first sublimation step to 35°C or below at a rate of 1.0-10.0 °C/h, and keeping it for 6-10 h after the temperature rises to 35°C;
S3.5, stoppering: after a pressure rise test is completed, introducing nitrogen, setting a pressure to 0.1-1.0 BarA, and performing a vacuum stoppering; after the stoppering, raising a plate layer, introducing a gas to restore a normal pressure, and obtaining a freeze-dried product.

2. The preparation method of the aminolevulinic acid hydrochloride freeze-dried formulation according to claim 1, wherein the step 1 further comprises adding a pharmaceutically acceptable excipient.

3. An aminolevulinic acid hydrochloride freeze-dried formulation prepared by the preparation method according to any one of claims 1-2.

4. The aminolevulinic acid hydrochloride freeze-dried formulation according to claim 3, wherein a moisture content is ≤1.0%, and a content of related substances is ≤1.0%; the contents above are all mass contents.

5. The aminolevulinic acid hydrochloride freeze-dried formulation according to claim 3, wherein the related substances comprise known impurities and unknown impurities, wherein a content of the known impurities in the aminolevulinic acid hydrochloride freeze-dried formulation does not exceed 0.1%, and the known impurities are 3,3'-(pyrazine-2,5-diyl)dipropanoic acid and mesityl oxide.

6. The aminolevulinic acid hydrochloride freeze-dried formulation according to claim 5, wherein a content of a component with a largest proportion of the unknown impurities in the aminolevulinic acid hydrochloride freeze-dried formulation does not exceed 0.2%.

7. The aminolevulinic acid hydrochloride freeze-dried formulation according to claim 3, wherein a content of the aminolevulinic acid hydrochloride is 95%-105% of a labeled amount.

8. The aminolevulinic acid hydrochloride freeze-dried formulation according to claim 3, wherein its pH is 2.0-3.0.

9. The aminolevulinic acid hydrochloride freeze-dried formulation according to claim 3, wherein its reconstitution time is ≤2 min.

10. The aminolevulinic acid hydrochloride freeze-dried formulation according to claim 3, wherein a freeze-dried formulation is a freeze-dried powder or a freeze-dried tablet.
